# EUROPEAN PATENT SPECIFICATION

(11) **EP 2 457 491 B1**
(45) Date of publication and mention of the grant of the patent: **15.10.2014**
(21) Application number: 10847977.5
(22) Date of filing: 29.10.2010
(51) Int. Cl.: A61B 1/00, A61B 1/005

(54) **ENDOSCOPE**
ENDOSKOP
ENDOSCOPE

(30) Priority: 15.03.2010 JP 2010057949
(43) Date of publication of application: 30.05.2012
(73) Proprietor: Olympus Medical Systems Corp., Tokyo 151-0072 (JP)
(72) Inventor: OUCHI, Naoya, Tokyo 151-0072 (JP)
(74) Representative: Schicker, Silvia
(86) International application number: PCT/JP2010/069330
(87) International publication number: WO 2011/114570

(56) References cited:
- EP-A1- 1 849 396
- JP-A- 4 117 939
- JP-A- 10 024 013
- JP-A- 10 201 703
- JP-A- 2002 143 084
- JP-A- 2003 093 330
- JP-A- 2003 093 330
- JP-A- 2007 054 125
- JP-U- H03 043 802
- JP-U- H04 013 101
- US-A- 5 179 935

## Description

### Technical Field

The present invention relates to an endoscope including an elongated insertion portion configured to be inserted into a subject and a bending portion provided to the insertion portion and including a first bending part and a second bending part.

### Background Art

In recent years, endoscopes have been widely used in medical fields and industrial fields. Endoscopes used in the medical fields are capable of observing an organ in a body cavity by inserting an elongated insertion portion into a body cavity as a subject and performing various kinds of treatment using, as needed, a treatment instrument inserted into a treatment instrument insertion channel provided to the endoscopes.

In addition, endoscopes used in the industrial fields are capable of performing examination such as observation of a flaw and corrosion of a region to be inspected in an object and various kinds of treatment by inserting an elongated insertion portion of the endoscope into an object such as a jet engine and a piping in a factory.

A configuration is well-known, in which a bending portion bendable in a plurality of directions is provided to an insertion portion of an endoscope. The bending portion improves the advanceability of the insertion portion in a flexure part in a conduit, and in addition, varies an observation direction of an observation optical system provided to a distal end portion which is located at the insertion portion on the more distal end side (hereinafter, only referred to as distal end side) in the insertion direction than the bending portion.

Generally, a bending portion provided to an insertion portion of an endoscope is configured to be bendable in four directions, namely, up, down, left and right directions, for example, by a plurality of bending pieces connected along the insertion direction of the insertion portion.

Furthermore, the bending portion is bendable in any of the up, down, left and right directions by pulling any of four wires which are inserted through the insertion portion and the distal ends of which are fixed to the bending piece located at the most distal side among the bending pieces, through an operation portion.

Incidentally, as described above, the bending portion is bent with a fixed amount of bending radius with the proximal end side of the bending portion in the insertion direction (hereinafter, simply referred to as proximal end side) as a starting point, by pulling operation of the wires. However, depending on a shape of inside a subject into which the insertion portion is inserted, there has been a desire for an endoscope having a configuration in which a bending radius of a bending portion can be freely varied in order to improve insertability of an insertion portion in the subject.

To this end, Japanese Patent Application Laid-Open Publication No. 2005-185526 discloses a configuration in which two shape-memory alloys, rigidity of which increases in accordance with the supply of current, are provided in the bending portion along the insertion direction, and the bending radius can be varied by the supply and non-supply of the current to the respective shape-memory alloys provided in the bending portion.

Specifically, the publication discloses that, when only an anterior part in the insertion direction of the bending portion is desired to be bent, current is supplied to the shape-memory alloy located at a posterior part in the insertion direction to increase the rigidity of the posterior part of the bending portion, thereby capable of bending only the anterior part according to the pulling of a wire, without bending the posterior part, in addition, when only the posterior part of the bending portion is desired to be bent, current is supplied to the shape-memory alloy located at the anterior part to increase the rigidity of the anterior part of the bending portion, thereby capable of bending only the posterior part according to the pulling of a wire, without bending the anterior part of the bending portion, and furthermore, when both the anterior part and the posterior part of the bending portion are desired to be bent, wire is pulled with the current not being supplied to both of the shape-memory alloys at the anterior part and the posterior part, thereby capable of bending the anterior part and the posterior part of the bending portion in the same direction.

However, according to the configuration recited in the Japanese Patent Application Laid-Open Publication No. 2005-185526, in order to increase rigidities of the respective shape-memory alloys provided to the bending portion, it is necessary to supply current to the respective shape-memory alloys. Therefore, there are such problems that a current supply apparatus has to be separately prepared and the configuration of the bending portion becomes complicated.

Therefore, there has been a desire for an endoscope having a configuration in which a bending radius of a bending portion can be easily varied with a simple configuration.

The present invention has been achieved in view of the above-described circumstances, and an object of the present invention is to provide an endoscope capable of easily varying a bending radius of a bending portion with a simple configuration and improving an insertability of an insertion portion.

Document EP 1 849 396 A1 discloses an endoscope flexible tube which exhibits flexibility to be inserted into a body cavity including a bending portion formed at a distal end side, a first flexible tube portion connected in series to a proximal end of the bending portion, and a second flexible tube portion connected in series to a proximal end of the first flexible tube portion. When the bending portion and the first flexible tube portion pass a flexed portion of the body cavity, a curvature radius of the first flexible tube portion which is passively bent under a predetermined force is set to be larger than a curvature radius of the bending portion in a maximum bent state.

### Disclosure of Invention

### Means for Solving the Problem

An endoscope according to the present invention includes the features defined by claim 1.

### Brief Description of the Drawings

FIG. 1 is a perspective view showing an appearance of an endoscope of a present embodiment.
FIG. 2 is a cross-sectional view of an insertion portion along the II-II line in FIG. 1.
FIG. 3 is a cross-sectional view of a coupling piece along the III-III line in FIG. 2.
FIG. 4 is a cross-sectional view of a second bending part along the IV-IV line in FIG. 2.
FIG. 5 is a cross-sectional view of a flexible tube portion along V-V line in FIG. 2.
FIG. 6 is an enlarged view of a region of the insertion portion surrounded by one-dot chain line VI in FIG. 1.
FIG. 7 is a view showing a state where only a first bending part in FIG. 6 is bent by pulling a first wire and a second wire.
FIG. 8 is a view showing a state where the second bending part is bent together with the first bending part in FIG. 6 in the same direction as a bending direction of the first bending part by pulling the first wire.
FIG. 9 is a partial cross-sectional view showing a configuration of an inside of a rigidity varying knob for the second bending part in FIG 1.
FIG 10 is an enlarged perspective view of an operation portion, which shows a modified example in which a lever for pulling the second wires is provided in the operation portion.
FIG 11 is a view showing a link mechanism for pulling the second wire provided in the operation portion in FIG 10.
FIG. 12 is a cross-sectional view of the link mechanism along the XII-XII line in FIG 11.

### Best Mode for Carrying Out the Invention

Hereinafter, an embodiment of the present invention will be described with reference to drawings. Note that each of the drawings is a pattern diagram, and care should be taken to the fact that the relationship between the thicknesses and widths of the respective members, a ratio of the thickness of a certain member to others, and the like are different from the actual sizes. It is needless to say that the relationship and the ratio among the dimensions are also different from one drawing to another.

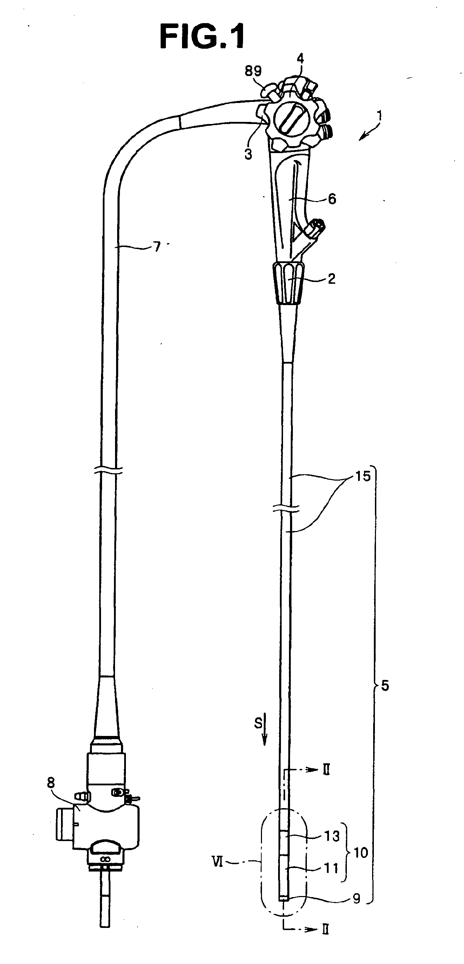

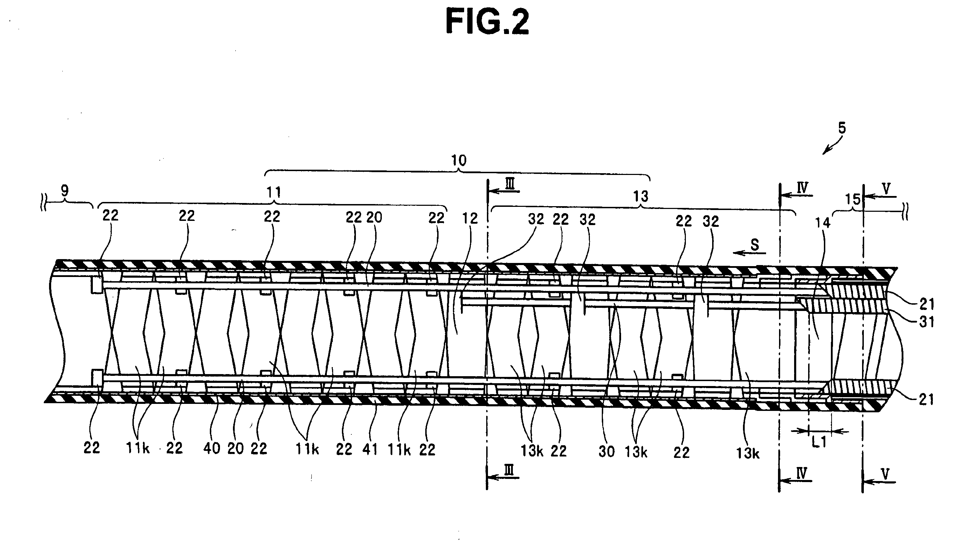

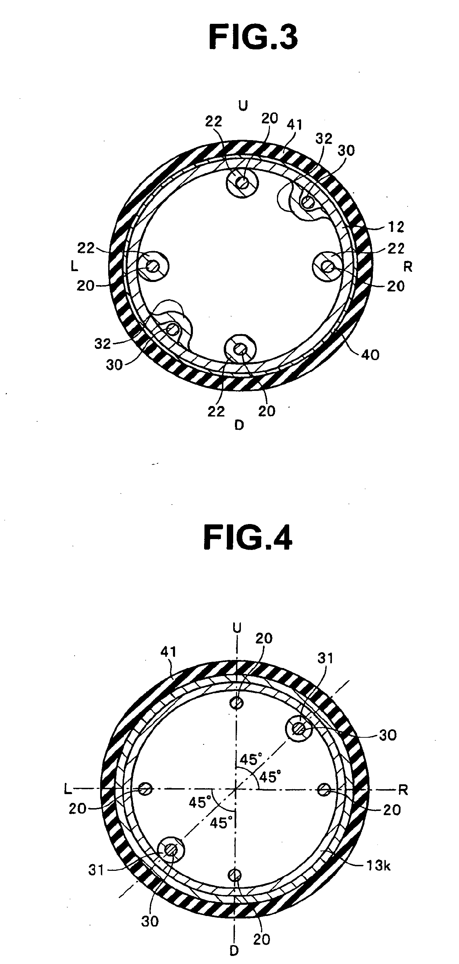

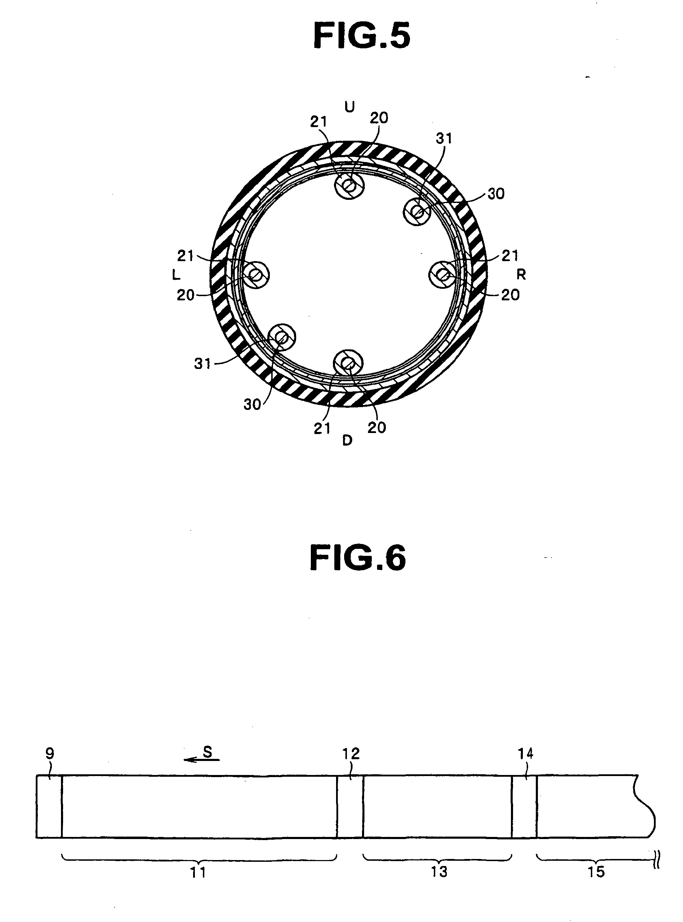

FIG. 1 is a perspective view showing an appearance of an endoscope of the present embodiment. FIG. 2 is a cross-sectional view of an insertion portion along the II-II line in FIG. 1. FIG. 3 is a cross-sectional view of a coupling piece along the III-III line in FIG. 2. FIG. 4 is a cross-sectional view of a second bending part along the IV-IV line in FIG. 2. FIG. 5 is a cross-sectional view of a flexible tube portion along the V-V line in FIG. 2.

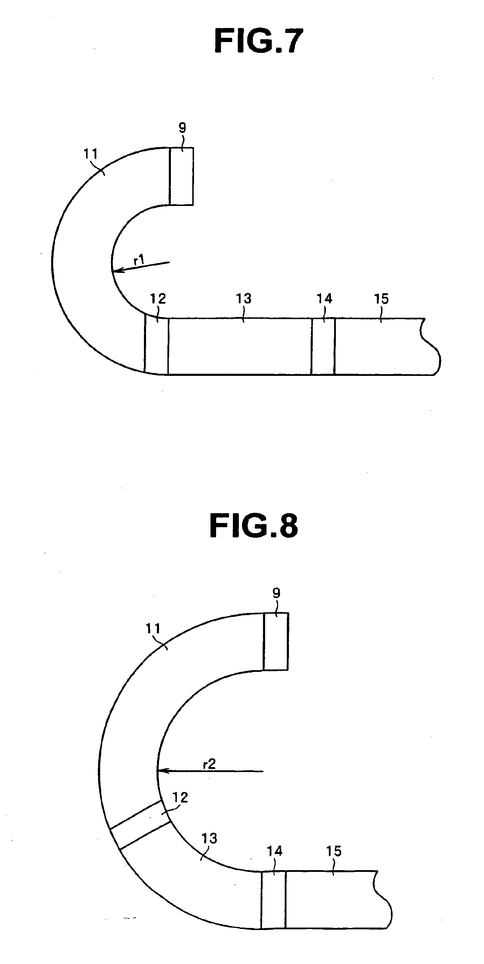

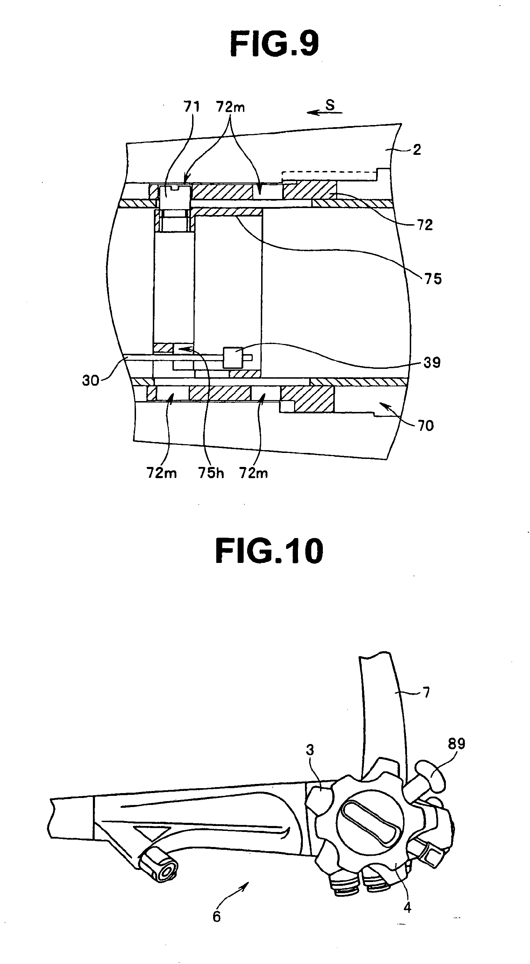

In addition, FIG. 6 is an enlarged view of a region of the insertion portion surrounded by one-dot chain line VI in FIG. 1. FIG. 7 is a view showing a state where only a first bending part in FIG. 6 is bent by pulling a first wire and a second wire. FIG. 8 is a view showing a state where the second bending part is bent together with the first bending part in FIG. 6 in the same direction as a bending direction of the first bending part by pulling the first wire. FIG. 9 is a partial cross-sectional view showing a configuration of an inside of a rigidity varying knob for the second bending part in FIG. 1.

As shown in FIG. 1, an endoscope 1 includes a main part configured by an insertion portion 5 configured to be inserted into a subject, an operation portion 6 provided in a linked manner on a proximal end side in an insertion direction S of the insertion portion 5, a universal cord 7 extended from the operation portion 6, and a connector 8 provided on an extension end of the universal cord 7. Note that the endoscope 1 is electrically connected to external devices such as a control device and an illumination device through the connector 8.

The operation portion 6 is provided with an up/down bending operation knob (hereinafter just referred to as a knob) 3 and a left/right bending operation knob (hereinafter just referred to as a knob) 4. In addition, a rigidity varying knob for a second bending part (hereinafter just referred to as a knob) 2 is provided between the operation portion 6 and the insertion portion 5.

As shown in FIGS. 1 and 6, the insertion portion 5 is constituted of a distal end portion 9, a bending portion 10, and a flexible tube portion 15 and formed in an elongated shape along the insertion direction S.

The distal end portion 9 includes inside thereof an image pickup unit, not shown, for observing inside a subject, an illumination unit for illuminating inside a subject, and the like.

Furthermore, the bending portion 10 includes a first bending part 11 which is independently bendable in four directions, i.e., up, down, left and right directions by the operation of the knob 3 or the knob 4, and a second bending part 13 whose rigidity is varied by the operation of the knob 2 and which is bendable together with the first bending part 11 by the operation of the knob 3 or knob 4 in four directions, namely, up, down, left and right directions so as to be bent in the same direction as a bending direction of the first bending part 11. Note that the bending directions of the first bending part 11 and the second bending part 13 are not limited to the four directions i.e., up, down, left and right directions, and the bending directions may be only two directions, namely, up and down directions or left and right directions.

In addition, as shown in FIGS. 2, 3 and 6, a coupling piece 12 for coupling the first bending part 11 and the second bending part 13 is provided between the first bending part 11 and the second bending part 13.

The flexible tube portion 15 is provided in a linked manner on the proximal end side of the bending portion 10, that is, the proximal end side of the second bending part 13. As shown in FIG. 2, the flexible tube portion 15 is provided in a linked manner on the proximal end side of the second bending part 13 through a coupling base 14.

Furthermore, as shown in FIG 2, inside the first bending part 11, a plurality of bending pieces 11k, which are bendable in four directions, i.e., up, down, left and right directions, are provided so as to be connected to each other along the insertion direction S. In addition, also inside the second bending part 13, a plurality of bending pieces 13k, which are bendable in four directions, i.e., up, down, left and right directions, are provided so as to be connected to each other along the insertion direction S.

As shown in FIGS. 2 to 5, the outer circumferences of the plurality of bending pieces 11k and 13k are covered with a braid 40, and the outer circumference of the braid 40 is covered with a bending rubber 41.

In addition, as shown in FIGS. 2 to 5, four first wires 20, which cause the first bending part 11 to independently bend in four directions, i.e., up, down, left and right directions, and which cause the second bending part 13 to bend together with the first bending direction in four directions, i.e., up, down, left and right directions so as to bend in the same direction as the bending direction of the first bending part 11, are inserted through the insertion portion 5 at positions different from each other by 90 degrees in the circumferential direction. Note that only two of the first wires 20 are shown in FIG. 2 for simplification of the drawing.

In addition, when the bending portion 10 is configured such that the first bending part 11 and the second bending part 13 are bent only in two directions, i.e., up and down directions, or left and right directions, it is sufficient that two first wires 20 are inserted through the insertion portion 5 at positions different from each other by 180 degrees in the circumferential direction, for example.

As shown in FIG. 2, each of the first wires 20 is supported in the bending portion 10 by wire receivers 22 provided to the respective bending pieces 11k and the respective bending pieces 13k such that the position in the circumferential direction is defined. Furthermore, as shown in FIG. 2, the distal end of each of the first wires 20 is fixed by brazing, for example, to the bending piece 11k located at the distal most in the insertion direction S among the plurality of bending pieces 11k.

Note that the proximal end of each of the first wires 20 is wound around a first pulley, not shown, which is rotatable by the knob 3 provided in the operation portion 6, or wound around a second pulley, not shown, which is rotatable by the knob 4.

In addition, as shown in FIGS. 2 and 5, in the flexible tube portion 15, the outer circumferences of the first wires 20 are covered with first guide pipes 21 distal ends of which are fixed to the coupling base 14. Note that the first wires 20 are inserted so as to be advanceable and retractable in the insertion direction S in the first guide pipes 21.

Note that, since the distal ends of the first guide pipes 21 are fixed to the coupling base 14, if either one of the two first wires 20 for up/down bending is pulled by the operation of the knob 3 or either one of the first wires 20 for left/right bending is pulled by the operation of the knob 4, when second wires 30 to be described later are in an unpulled state, the first bending part 11 and the second bending part 13 are bent in either the up direction or the down direction, or either the left direction or the right direction, with the distal ends of the first guide pipes 21 as a starting point, as shown in FIG. 8. That is, the bending portion 10 is bent with a bending radius r2, as shown in FIG. 8.

As shown in FIGS. 2 to 5, two second wires 30, which cause the second bending part 13 to be rigid when pulled, are inserted in the insertion portion 5 so as to be located at positions different from each other by 180 degrees, for example, in the circumferential direction and displaced from the positions of the first wires 20 and the first guide pipes 21 by 40 to 50 degrees in the circumferential direction, for example. Note that more than two, that is, three or four second wires 30 may be inserted through the insertion portion 5.

Note that only one of the two second wires 30 is shown in FIG. 2 for simplicity sake of the drawing.

The second wires 30 are supported in the bending pieces 13k of the second bending part 13 by string guides 32 pressed onto the respective bending pieces 13k such that the positions in the circumferential direction are defined. The distal ends of the second wires are fixed to the coupling piece 12 by blazing, for example, as shown in FIG. 2.

Note that the second wires 30 are not limited to be supported by the string guides 32, but may be supported in the bending pieces 13k by wire receivers similarly as the first wires 20. However, the string guides 32 are smaller members than the wire receivers. Therefore, if the string guides 32 are used, it is possible to decrease the filling rate of the internal components in the second bending part 13 through which the four first wires 20 and the two second wires 30 are inserted.

The proximal ends of the two second wires 30 are fixed to a pulling mechanism 70 provided in the knob 2, as shown in FIG 9.

Specifically, as shown in FIG. 9, the pulling mechanism 70 includes a main part configured by a moving ring 75 with which stoppers 39 provided on the respective proximal ends of the two second wires 30 are engageable, a cam ring 72 which is rotatably fitted on the outer circumference of the moving ring 75 and which has a cam groove 72m into which a cam pin 71 fixed on the outer circumferential surface of the moving ring 75 is fitted, and the knob 2 fitted on the outer circumference of the cam ring 72. Note that only one of the two second wires 30 is shown in FIG. 9 for simplification of the drawing.

According to such a configuration of the pulling mechanism 70, when the knob 2 is rotated in one direction by the operator, also the cam ring 72 is rotated in one direction, which causes the cam pin 71 to move in the cam groove 72m, and as a result, the moving ring 75 moves rearward in the insertion direction S.

As a result, the stoppers 39 provided on the respective proximal ends of the two second wires 30 are fitted into fitting holes 75h formed at the moving ring 75 and the stoppers 39 are engaged in the fitting holes 75h, which causes the two second wires 30 are pulled rearward in the insertion direction S at the same time.

That is, the two second wires 30 are configured to be pulled rearward in the insertion direction S at the same time by the pulling mechanism 70, when the knob 2 is rotated in one direction. Note that the above-described pulling mechanism works in the same way even in a case where three or four second wires 30 are provided.

Furthermore, the outer circumferences of the second wires 30 are covered with second guide pipes 31 in the flexible tube portion 15, as shown in FIGS. 2, 4 and 5. The distal ends of the second guide pipes 31 are fixed to the coupling base 14 located at the rear of the second bending part 13. Note that the second wires 30 are inserted in the second guide pipes 31 so as to be advanceable and retractable in the insertion direction S.

Furthermore, as shown in FIG. 2, the distal ends of the second guide pipes 31 are fixed at positions protruded forward in the insertion direction S from the distal ends of the first guide pipes 21 by a predetermined length L1. Such a configuration is for preventing the filling rate of the internal components in the second bending part 13 from suddenly changing along the insertion direction S.

Specifically, if the distal ends of the first guide pipes 21 are fixed at the same positions in the insertion direction S as those of the second guide pipes 31, the total number of the guide pipes is suddenly changed toward forward in the insertion direction S from six including the four first guide pipes 21 plus the two second guide pipes 31 to zero, that is, the filling rate of the internal components in the second bending part 13 suddenly changes.

However, in the present embodiment, the distal ends of the second guide pipes 31 protrude forward more than the distal ends the first guide pipes 21 by the predetermined length L1. Therefore, the total number of the guide pipes changes in a step-by-step manner in the second bending part 13 toward forward in the insertion direction S, from six including the four first guide pipes 21 plus two second guide pipes 31 to two including only the two second guide pipes 31, and then to zero including no guide pipe. In other words, the filling rate of the internal components in the second bending part 13 can be changed in a step-by-step manner.

Note that a sudden change in the filling rate of the internal components in the second bending part 13 is unfavorable, because the internal components in the bending portion 10 normally slidingly move forward and backward along the insertion direction S in accordance with bending of the bending portion 10, and if there are a large number of internal components in the bending portion, the sliding resistance increases, which causes difficulty in movement of the internal components. Therefore, if the filling rate of the internal components suddenly changes along the insertion direction S, the internal components can easily move in some part of the bending portion but can hardly move in other part of the bending portion, which is likely to cause damage on the internal components.

Therefore, if the filling rate of the internal components changes in a step-by-step manner as in the present embodiment, it is easy for the internal components in the bending portion 10 to move smoothly in accordance with bending operation. As a result, it is possible to protect the internal components.

Returning to FIG. 2, the distal ends of the second wires 30 are fixed to the coupling piece 12. When the two second wires 30 are pulled by the operation of the knob 2, the two second wires 30 stretch with respect to the insertion direction S, because the position of the coupling piece 12 is fixed. Therefore, the plurality of bending pieces 13k are compressed in the insertion direction S. As a result, the second bending part 13 becomes rigid.

In the state where the second bending part 13 is rigid, if either one of the two first wires 20 for up/down bending is pulled by the operation of the knob 3 or either one of the two first wires 20 for left/right bending is pulled by the operation of the knob 4, only the first bending part 11 bends in either the up direction or the down direction, or either the left direction or the right direction, with the distal ends of the second wires 30 as a starting point, as shown in FIG. 7. That is, as shown in FIG. 7, the bending portion 10 bends with the bending radius r1 smaller than the bending radius r2.

Thus, in the present embodiment, the distal ends of the first wires 20 are fixed to the bending piece 11k located forward-most in the first bending part 11, the distal ends of the first guide pipes 21 covering the outer circumferences of the first wires 20 are fixed to the coupling piece 14, and the distal ends of the second wires 30 are fixed to the coupling piece 12.

In addition, when the second wires 30 are pulled by the operation of the knob 2, the second bending part 13 becomes rigid, and only the first bending part 11 is bendable in the up/down direction, or the left/right direction with the distal ends of the second wires 30 as a starting point according to the pulling operation of one of the first wires 20 by the operation of the knob 3 or the knob 4. In addition, when the second wires 30 are in an unpulled state, the first bending part 11 and the second bending part 13 are bendable in the up/down direction, or the left/right direction with the distal ends of the first guide pipes 21 as a starting point.

According to such a configuration, the bending radius can be changed by pulling or not pulling the second wires 30, that is, by causing or not causing the second bending part 13 to be rigid. Therefore, it is possible to change the bending radius of the bending portion 10 with a simple configuration, and it is possible to provide the endoscope 1 capable of improving insertability of the insertion portion 5.

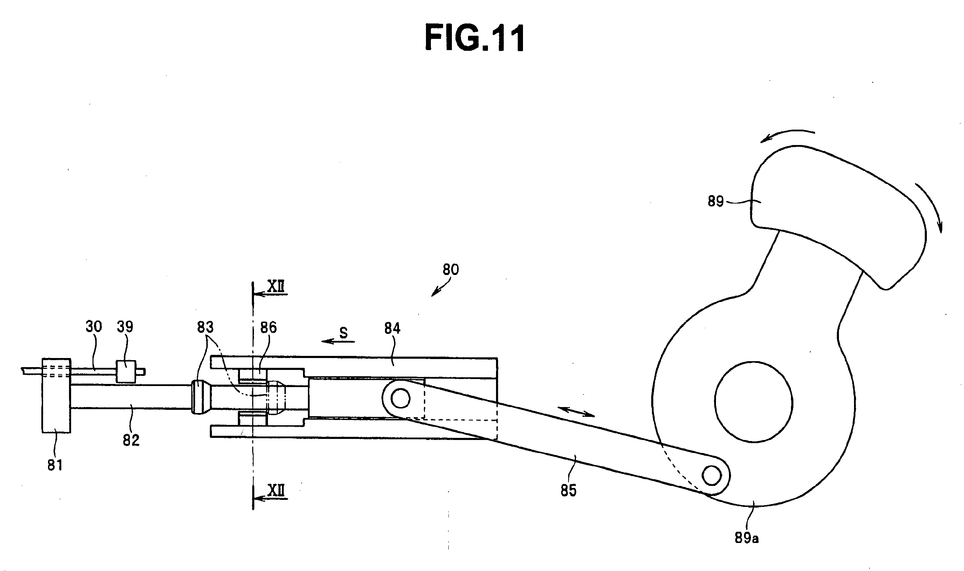

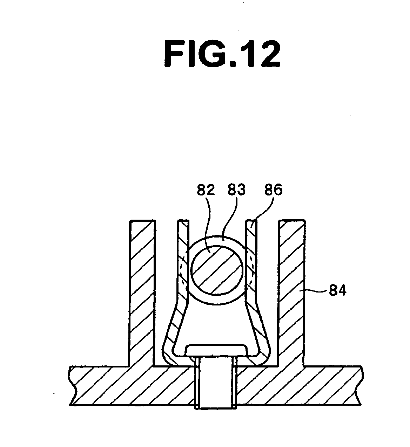

Note that modified examples are shown below with reference to FIGS. 10 to 12. FIG. 10 is an enlarged perspective view of the operation portion, which shows a modified example in which a lever for pulling the second wires is provided in the operation portion. FIG. 11 is a view showing a link mechanism for pulling the second wires provided in the operation portion in FIG. 10. FIG. 12 is a cross-sectional view of the link mechanism along the XII-XII line in FIG. 11.

In the above-described present embodiment, the two second wires 30 are pulled by using the pulling mechanism 70 including the moving ring 75 and the cam ring 72 by rotating the knob 2 in one direction.

However, the pulling mechanism is not limited to the above configuration, and may be configured by using a link mechanism 80 shown in FIGS. 10 to 12.

Specifically, as shown in FIG. 10, the operation portion 6 is provided with a lever 89 which is operated when the second two wires 30 are pulled. As shown in FIG. 11, a rotation axis 89a of the lever 89 is jointed with a rear end of a link 85.

As shown in FIG. 11, the distal end of the link 85 is rotatably connected to the rear end of a rod 82 inserted in a guide member 84, and at a distal end of the rod 82, an engaging member 81 is fixed.

The proximal end sides of the second wires 30 are penetrated through the engaging member 81 along the insertion direction S, and the engaging member 81 is configured to be engageable with the stoppers 39 provided at the proximal ends of the second wires 30. Note that only one of the two second wires 30 is shown in FIG. 11 for the simplification of the drawing.

Therefore, when the lever 89 is rotated by the operator, the rod 82 is pulled rearward of the insertion direction S in the guide member 84 by the link 85. At this time, the stoppers 39 of the second wires 30 are engaged with the engaging member 81, thereby causing the second wires 30 to be pulled rearward together with the rod 82.

Note that a projection portion 83 is provided at a halfway position in the insertion direction S of the rod 82, as shown in FIG 11. When the lever 89 is rotated to cause the rod 82 to be pulled rearward by the link 85, the projection portion 83 is fixed to a plate spring 86 shown in FIG. 12 which is provided on the inner circumferential surface on the distal end side of the guide member 84, by snap fit as shown by two-dot-chain lines in FIG. 11. This allows the position of the rod 82 to be fixed, and thereby the pulled state of the second wires 30 is fixed.

Thus, also in the case where the link mechanism 80 is used as the pulling mechanism for the second wires 30, it is possible to obtain the same effects as those of the present embodiment.

## Claims

1. An endoscope (1) comprising:
an elongated insertion portion (5) configured to be inserted in a subject;
a bending portion (10) provided at the insertion portion (5), the bending portion (10) including a first bending part (11) which is independently bendable and a second bending part (13) which is provided in a linked manner on a proximal end side of the first bending part (11) and which is bendable together with the first bending part (11) in the same direction as a bending direction of the first bending part (11); and
a flexible tube portion (15) provided in a linked manner on a proximal end side of the bending portion (10) at the insertion portion (5) through a coupling base (14); the endoscope (1) further comprising:
one or more first wires (20) inserted in the insertion portion (5), each of the one or more first wires (20) having a distal end fixed to a distal end of the first bending part (11);
one or more first guide pipes (21) through which the one or more first wires (20) are inserted so as to be advanceable and retractable with respect to an insertion direction (S) of the insertion portion (5), each of the one or more first guide pipes (21) having a distal end fixed between the bending portion (10) and the flexible tube portion (15) to the coupling base (14);
one or more second wires (30) inserted in the insertion portion (5), each of the one or more second wires (30) having a distal end fixed between the first bending part (11) and the second bending part (13), the one or more second wires (30) causing the second bending part (13) to be rigid when pulled; and
one or more second guide pipes (31) through which the one or more second wires (30) are inserted so as to be advanceable and retractable with respect to the insertion direction (S), each of the one or more second guide pipes (31) having a distal end fixed to the coupling base (14) at a position protruding forward in the insertion direction (S) from the distal end of each of the one or more first guide pipes (21) by a predetermined length (L1), wherein
when any of the one or more first wires (20) is pulled in a state where the one or more second wires (30) are pulled and the second bending part (13) is caused to be rigid, the bending portion (10) is bent such that only the first bending part (11) is bent with the distal end of each of the one or more second wires (30) as a starting point, and
when any of the one or more first wires (20) is pulled in a state where the one or more second wires (30) are not pulled and the second bending part (13) is not caused to be rigid, the bending portion (10) is bent such that the second bending part (13) is bent together with the first bending part (11) in the same direction, with the distal end of each of the one or more first guide pipes (21) as a starting point.

2. The endoscope (1) according to claim 1, wherein the one or more first wires (20) and first guide pipes (21) are inserted into the insertion portion (5) in such a manner that two first wires (20) and first guide pipes (21) are located at positions displaced with respect to each other by 180 degrees in a circumferential direction such that the positions correspond to up and down directions, respectively, or left and right directions, respectively, among bending directions of the bending portion (10), or in such a manner that four first wires (20) and first guide pipes (21) are located at positions displaced with respect to each other by 90 degrees in the circumferential direction such that the positions correspond to the up, down, left and right directions, respectively.

3. The endoscope (1) according to claim 2, wherein the one or more second wires (30) and second guide pipes (31) are inserted in the insertion portion (5) so as to be located at positions displaced by 40 to 50 degrees in the circumferential direction with respect to the first wires (20) and first guide pipes (21).

## Patentansprüche

1. Endoskop (1), das umfasst:
einen langestreckten Einführabschnitt (5), der dazu eingerichtet ist, in ein Subjekt eingeführt zu werden;
einen Biegeabschnitt (10), der an dem Einführabschnitt (5) vorgesehen ist, wobei der Biegeabschnitt (10) ein erstes Biegeteil (11), das unabhängig biegbar ist, und ein zweites Biegeteil (13) umfasst, das in einer verbundenen Art und Weise an einer proximalen Endseite des ersten Biegeteils (11) vorgesehen ist und das zusammen mit dem ersten Biegeteil (11) in die gleiche Richtung biegbar ist, wie eine Biegerichtung des ersten Biegeteils (11); und
einen flexiblen Röhrenabschnitt (15), der durch eine Verbindungsbasis (14) in einer verbundenen Art und Weise an einer proximalen Endseite des Biegeabschnitts (10) an dem Einführabschnitt (5) vorgesehen ist;
wobei das Endoskop (1) ferner umfasst:
einen ersten Draht oder mehrere erste Drähte (20), der/die in den Einführabschnitt (5) eingeführt ist/sind, wobei der erste Draht oder jeder der mehreren ersten Drähte (20) ein distales Ende hat, das an einem distalen Ende des ersten Biegeteils (11) angebracht ist;
ein erstes Führungsrohr oder mehrere erste Führungsrohre (21), durch das/die der erste Draht oder die mehreren ersten Drähte (20) eingeführt ist/sind, so dass er/sie in Bezug auf eine Einführrichtung (S) des Einführabschnitts (5) vorschiebbar oder zurückziehbar ist/sind, wobei das erste Führungsrohr oder jedes der mehreren ersten Führungsrohre (21) ein distales Ende hat, das zwischen dem Biegeabschnitt (10) und dem flexiblen Röhrenabschnitt (15) an der Verbindungsbasis (14) angebracht ist;
einen zweiten Draht oder mehrere zweite Drähte (30), der/die in den Einführabschnitt (5) eingeführt ist/sind, wobei der zweite Draht oder jeder der mehreren zweiten Drähte (30) ein distales Ende hat, das zwischen dem ersten Biegeteil (11) und dem zweiten Biegeteil (13) angebracht ist, wobei der zweite Draht oder die mehreren zweiten Drähte (30) den zweiten Biegeabschnitt (13) veranlassen, steif zu sein, wenn er/sie gezogen wird/werden; und
ein zweites Führungsrohr oder mehrere zweite Führungsrohre (31), durch das/die der zweite Draht oder die mehreren zweiten Drähte (30) eingeführt ist/sind, so dass er/sie in Bezug auf die Einführrichtung (S) vorschiebbar oder zurückziehbar ist/sind, wobei das zweite Führungsrohr oder jedes der mehreren zweiten Führungsrohren (31) ein distales Ende hat, das an einer Position, die um eine vorbestimmte Länge (L1) in der Einführrichtung (S) von dem distalen Ende des ersten Führungsrohrs oder jedes der mehreren ersten Führungsrohre (21) vorsteht, an der Verbindungsbasis (14) angebracht ist, wobei
wenn der erste Draht oder einer der mehreren ersten Drähte (20) in einen Zustand gezogen wird, in dem der zweite Draht oder die mehreren zweiten Drähte (30) gezogen ist/sind und das zweite Biegeteil (13) veranlasst ist, steif zu sein, der Biegeabschnitt (10) so gebogen wird, dass nur das erste Biegeteil (11) mit dem distalen Ende des zweiten Drahts oder jedem der mehreren zweite Drähte (30) als Startpunkt gebogen wird, und
wenn der erste Draht oder einer der mehreren ersten Drähte (20) in einen Zustand gezogen wird, in dem der zweite Draht oder die mehreren zweiten Drähte (30) nicht gezogen ist/sind und das zweite Biegeteil (13) nicht veranlasst ist, steif zu sein, der Biegeabschnitt (10) so gebogen wird, dass das zweite Biegeteil (13) gemeinsam mit dem ersten Biegeteil (11) in die gleiche Richtung mit dem distalen Ende des ersten Führungsrohrs oder jedem der mehreren ersten Führungsrohre (21) als Startpunkt gebogen wird.

2. Endoskop (1) gemäß Anspruch 1, wobei der erste Draht oder die mehreren ersten Drähte (20) und das erste Führungsrohr oder die mehreren ersten Führungsrohre (21) in einer Art und Weise in den Einführabschnitt (5) eingeführt sind, dass zwei erste Drähte (20) und erste Führungsrohre (21) in Positionen angeordnet sind, die bezüglich einander um 180 Grad in einer Umfangsrichtung versetzt sind, so dass die Positionen Aufwärts- beziehungsweise Abwärtsrichtungen oder Links- beziehungsweise Rechtsrichtungen unter Biegerichtungen des Biegeabschnitts (10) entsprechen, oder in einer Art und Weise, dass vier erste Drähte (20) und erste Führungsrohre (21) in Positionen angeordnet sind, die bezüglich einander um 90 Grad in der Umfangsrichtung versetzt sind, so dass die Positionen den Aufwärts-, Abwärts-, Links- beziehungsweise Rechtsrichtungen entsprechen.

3. Endoskop (1) gemäß Anspruch 2, wobei der zweite Draht oder die mehreren zweite Drähte (30) und das zweite Führungsrohr oder die mehreren zweiten Führungsrohre (31) so in den Einführabschnitt (5) eingeführt sind, dass sie an Positionen angeordnet sind, die bezüglich der ersten Drähte (20) und der ersten Führungsrohre (21) um 40 bis 50 Grad in der Umfangsrichtung versetzt sind.

## Revendications

1. Endoscope (1) comprenant :
une partie d'insertion (5) allongée configurée pour être insérée dans un sujet ;
une partie de cintrage (10) prévue au niveau de la partie d'insertion (5), la partie de cintrage (10) comprenant une première partie de cintrage (11) qui peut être cintrée indépendamment et une deuxième partie de cintrage (13) qui est prévue de manière reliée sur un côté d'extrémité proximale de la première partie de cintrage (11) et qui peut être cintrée conjointement avec la première partie de cintrage (11) dans la même direction qu'une direction de cintrage de la première partie de cintrage (11) ; et
une partie de tube flexible (15) prévue de manière reliée sur un côté d'extrémité proximale de la partie de cintrage (10) au niveau de la partie d'insertion (5) par l'intermédiaire d'une base de couplage (14) ;
l'endoscope (1) comprenant en outre :
un ou plusieurs premiers câbles (20) insérés dans la partie d'insertion (5), chacun parmi le ou les plusieurs premiers câble (20) comportant une extrémité distale fixée à une extrémité distale de la première partie de cintrage (11) ;
un ou plusieurs premiers tuyaux de guidage (21) à travers lesquels le ou les plusieurs premiers câbles (20) sont insérés de façon à pouvoir être avancés et rétractés par rapport à une direction d'insertion (S) de la partie d'insertion (5), chacun parmi le ou les plusieurs premiers tuyaux de guidage (21) comportant une extrémité distale fixée entre la partie de cintrage (10) et la partie de tube flexible (15) sur la base de couplage (14) ;
un ou plusieurs deuxièmes câbles (30) insérés dans la partie d'insertion (5), chacun parmi le ou les plusieurs deuxièmes câbles (30) comportant une extrémité distale fixée entre la première partie de cintrage (11) et la deuxième partie de cintrage (13), le ou les plusieurs deuxièmes câbles (30) amenant la deuxième partie de cintrage (13) à être rigide en étant tirés ; et
un ou plusieurs deuxièmes tuyaux de guidage (31) à travers lesquels le ou les plusieurs deuxièmes câbles (30) sont insérés de façon à pouvoir être avancés et rétractés par rapport à la direction d'insertion (S), chacun parmi le ou les plusieurs deuxièmes tuyaux de guidage (31) comportant une extrémité distale fixée à la base de couplage (14) au niveau d'une position faisant saillie vers l'avant dans la direction d'insertion (S) depuis l'extrémité distale de chacun parmi le ou les plusieurs premiers tuyaux de guidage (21) d'une longueur prédéterminée (L1), dans lequel
lorsque l'un quelconque parmi le ou les plusieurs premiers câbles (20) est tiré dans un état dans lequel le ou les plusieurs deuxièmes câbles (30) sont tirés et que la deuxième partie de cintrage (13) est amenée à être rigide, la partie de cintrage (10) est cintrée d'une manière telle que seule la première partie de cintrage (11) est cintrée avec l'extrémité distale de chacun parmi le ou les plusieurs deuxièmes câbles (30) comme point de départ, et
lorsque l'un quelconque parmi le ou les plusieurs premiers câbles (20) est tiré dans un état dans lequel le ou les plusieurs deuxièmes câbles (30) ne sont pas tirés et que la deuxième partie de cintrage (13) n'est pas amenée à être rigide, la partie de cintrage (10) est cintrée d'une manière telle que la deuxième partie de cintrage (13) est cintrée conjointement avec la première partie de cintrage (11) dans la même direction, avec l'extrémité distale de chacun parmi le ou les plusieurs premiers tuyaux de guidage (21) comme point de départ.

2. Endoscope (1) selon la revendication 1, dans lequel le ou les plusieurs premiers câbles (20) et premiers tuyaux de guidage (21) sont insérés dans la partie d'insertion (5) d'une manière telle que deux premiers câbles (20) et les premiers tuyaux de guidage (21) sont placés à des positions déplacés les uns par rapport aux autres de 180 degrés dans une direction circonférentielle d'une manière telle que les positions correspondent aux directions vers le haut et vers le bas, respectivement, ou aux directions vers la gauche et vers la droite, respectivement, parmi les directions de cintrage de la partie de cintrage (10), ou d'une manière telle que quatre premiers câbles (20) et premiers tuyaux de guidage (21) sont placés à des positions déplacés les uns par rapport aux autres de 90 degrés dans la direction circonférentielle d'une manière telle que les positions correspondent aux directions vers le haut, vers le bas, vers la gauche et vers la droite, respectivement.

3. Endoscope (1) selon la revendication 2, dans lequel le ou les plusieurs deuxièmes câbles (30) et deuxièmes tuyaux de guidage (31) sont insérés dans la partie d'insertion (5) de façon à être placés à des positions déplacés de 40 à 50 degrés dans la direction circonférentielle par rapport aux premiers câbles (20) et aux premiers tuyaux de guidage (21).
